# EUROPÄISCHE PATENTSCHRIFT

(11) **EP 1 242 388 B1**
(45) Veröffentlichungstag und Bekanntmachung des Hinweises auf die Patenterteilung: **23.07.2003**
(21) Anmeldenummer: 00991158.7
(22) Anmeldetag: 08.12.2000
(51) Int. Cl.: C07D 249/12

(54) **VERFAHREN ZUR HERSTELLUNG EINES TRIAZOLINTHION-DERIVATES**
METHOD FOR PRODUCTION OF A TRIAZOLINETHIONE DERIVATIVE
PROCEDE POUR PRODUIRE UN DERIVE DE TRIAZOLINE-THIONE

(30) Priorität: 21.12.1999 DE 19961603
(43) Veröffentlichungstag der Anmeldung: 25.09.2002
(73) Patentinhaber: Bayer CropScience AG, 40789 Monheim (DE)
(72) Erfinder: HUPPERTS, Achim, 40477 Düsseldorf (DE); RUTHER, Michael, 40764 Langenfeld (DE); JAUTELAT, Manfred, 51399 Burscheid (DE)
(86) Internationale Anmeldenummer: EP0012494
(87) Internationale Veröffentlichungsnummer: WO01046158

(56) Entgegenhaltungen:
- EP-A- 0 297 345
- WO-A-99/18086
- WO-A-99/18087
- WO-A-99/18088
- DE-A- 4 030 039
- I ARAI: "BULLETIN OF THE CHEMICAL SOCIETY OF JAPAN,JP,JAPAN PUBLICATIONS TRADING CO. TOKYO" BULLETIN OF THE CHEMICAL SOCIETY OF JAPAN,JP,JAPAN PUBLICATIONS TRADING CO. TOKYO, Bd. 46, Nr. 7, 1. Juli 1973 (1973-07-01), Seiten 2215-2218, XP002087557 ISSN: 0009-2673
- DATABASE CHEMABS [Online] CHEMICAL ABSTRACTS SERVICE, COLUMBUS, OHIO, US; MOZOLIS, V. ET AL: "Synthesis of disulfides and sulfides containing sulfo groups" retrieved from STN Database accession no. 87:101885 XP002167238 & LIET. TSR MOKSLU AKAD. DARB., SER. B (1977), (2), 23-7 ,

## Beschreibung

Die vorliegende Erfindung betrifft ein neues Verfahren zur Herstellung von 2-(1-Chlor-cycloprop- 1-yl)-1-(2-chlor-phenyl)-3-(4,5-dihydro-1,2,4-triazol-5-thiono-1-yl)-propan-2-ol, das als Wirkstoff mit mikrobiziden, insbesondere fungiziden Eigenschaften bekannt ist.

Es ist bereits bekannt, dass sich 2-(1-Chlor-cycloprop-1-yl)-1-(2-chlor-phenyl)-3-(4,5-dihydro-1,2,4-triazol-5-thiono-1-yl)-propan-2-ol herstellen lässt, indem man zunächst 3-Chlor-2-(1-chlor-cycloprop-1-yl)-1-(2-chlor-phenyl)- propan-2-ol mit Hydrazinhydrat, gegebenenfalls in Gegenwart eines inerten organischen Lösungsmittels, wie Alkohol, Ether oder Nitril, umsetzt, das dabei entstehende 2-(1-Chlor-cycloprop-1-yl)-3-(2-chlor-phenyl)-2-hydroxy-propyl-1-hydrazin dann mit Formaldehyd und Alkalimetall- oder Ammonium-thiocyanat zur Reaktion bringt und schließlich das so erhaltene 2-(1-Chlor-cycloprop-1-yl)-1-(2-chlor-phenyl)-2-hydroxy-3-(1,2,4-triazolidin-5-thiono-1-yl)-propan mit Sauerstoff in Gegenwart von Schwefel und Kaliumhydroxid umsetzt (vgl. WO 99-18 087). Die Reaktionsfolge kann durch das nachstehende Formelschema veranschaulicht werden:

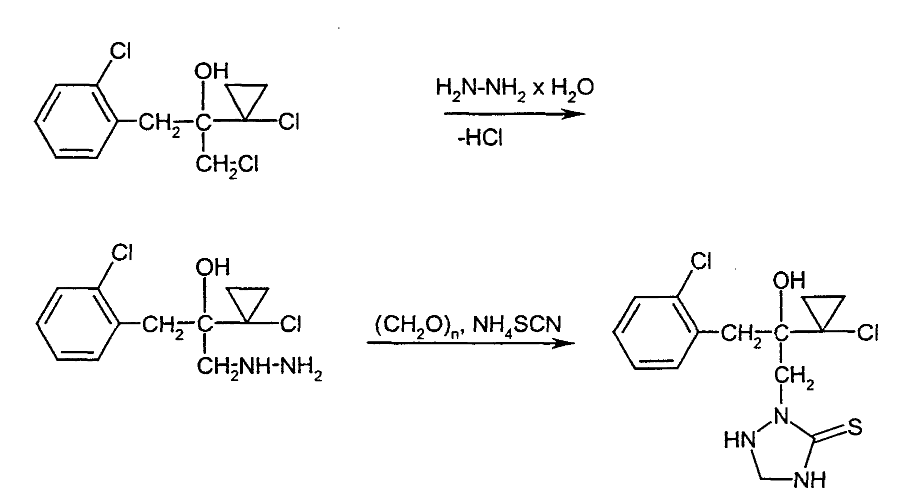

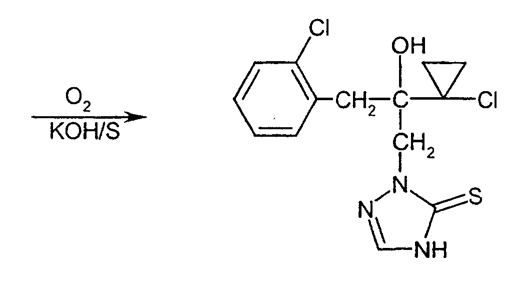

Nachteilig an diesem Verfahren ist, dass die in der ersten Stufe entstehende Hydrazin-Verbindung in freiem Zustand relativ instabil ist. Ungünstig ist auch, dass sich im Verlauf der mehrstufigen Synthese unerwünschte Nebenprodukte bilden und die Ausbeute für eine Herstellung im technischen Maßstab verhältnismäßig niedrig ist. Beeinträchtigend ist schließlich auch, dass bei der Durchführung der dritten Stufe eine störende Überoxidation stattfinden kann, wodurch Schwefel aus dem Zielprodukt abgespalten wird.

Es wurde nun gefunden, dass sich 2-(1-Chlor-cycloprop-1-yl)-1-(2-chlor-phenyl)-3-(4,5-dihydro-1,2,4-triazol-5-thiono-1-yl)-propan-2-ol der Formel 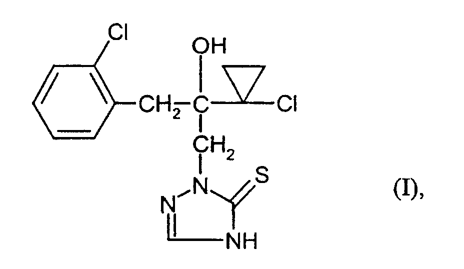 herstellen lässt, indem man
a) in einer ersten Stufe 2-(1-Chlor-cycloprop-1-yl)-2-(2'-chlor-benzyl)-oxiran der Formel 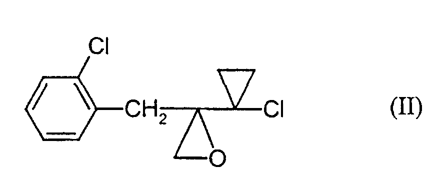 zunächst mit Hydrazinhydrat in Gegenwart von aromatischem Kohlenwasserstoff, gegebenenfalls im Gemisch mit Acetonitril, umsetzt und dann Chlorwasserstoff einleitet, oder mit wässriger Salzsäure extrahiert,
b) danach in einer zweiten Stufe das entstandene 2-(1-Chlor-cycloprop-1-yl)-3-(2-chlor-phenyl)-2-hydroxy-propyl-1-hydrazin-hydrochlorid der Formel 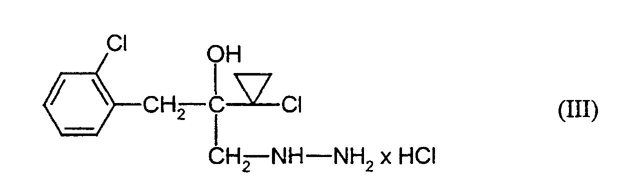 mit Alkalimetall-hydroxid in Gegenwart von Wasser sowie in Gegenwart von aromatischem Kohlenwasserstoff, gegebenenfalls im Gemisch mit einem niederen Alkohol, oder in Gegenwart von Alkylcarbonsäurealkylester behandelt und dann nacheinander mit Formaldehyd und Thiocyanat der Formel

   X-SCN (IV),

   in welcher
   - X: für Natrium, Kalium oder Ammonium steht,
   in Gegenwart von Wasser sowie in Gegenwart von aromatischem Kohlenwasserstoff, gegebenenfalls im Gemisch mit einem niederen Alkohol, oder in Gegenwart von Alkylcarbonsäurealkylester sowie gegebenenfalls in Gegenwart eines Katalysators umsetzt und
c) anschließend in einer dritten Stufe das entstandene 2-(1-Chlor-cycloprop-1-yl)-1-(2-chlor-phenyl)-2-hydroxy-3-(1,2,4-triazolidin-5-thiono-1-yl)-propan der Formel 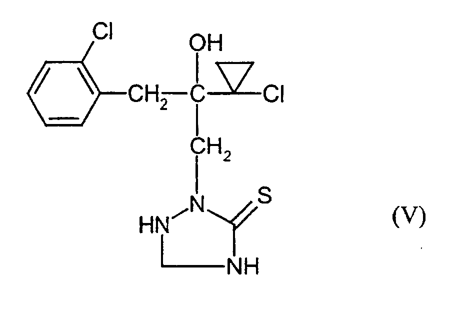 mit Eisen (III)-chlorid in Gegenwart von wässriger Salzsäure sowie in Gegenwart eines inerten organischen Verdünnungsmittels umsetzt.

Es ist als ausgesprochen überraschend zu bezeichnen, dass sich das Triazolinthion-Derivat der Formel (I) nach dem erfindungsgemäßen Verfahren in höheren Ausbeuten herstellen lässt als nach den bisher bekannten Methoden. Unerwartet ist auch, dass im Verlauf der mehrstufigen Synthese praktisch keine störenden Nebenreaktionen auftreten.

Das erfindungsgemäße Verfahren zeichnet sich durch eine Reihe von Vorteilen aus. So ermöglicht es, wie schon erwähnt, die Synthese des Triazolinthion-Derivates der Formel (I) in hoher Ausbeute. Günstig ist auch, dass die benötigten Ausgangsstoffe und Reaktionskomponenten einfach herstellbar und auch in größeren Mengen verfügbar sind. Ein weiterer Vorteil besteht darin, dass die Durchführung der einzelnen Reaktionsschritte und die Isolierung der Reaktionsprodukte keinerlei Schwierigkeiten bereitet. Schließlich sei auch erwähnt, dass sich das Hydrazinhydrochlorid-Derivat der Formel (III) im Gegensatz zu der entsprechenden Hydrazin-Verbindung ohne Stabilitätsprobleme handhaben lässt und dass in der letzten Stufe eine Überoxidation vermieden werden kann.

Verwendet man bei der Durchführung der zweiten Stufe Natriumhydroxid als Neutralisationsmittel und Natriumthiocyanat sowie Formalinlösung als Reaktionskomponenten, so kann der Verlauf des erfindungsgemäßen Verfahrens durch das folgende Formelschema veranschaulicht werden:

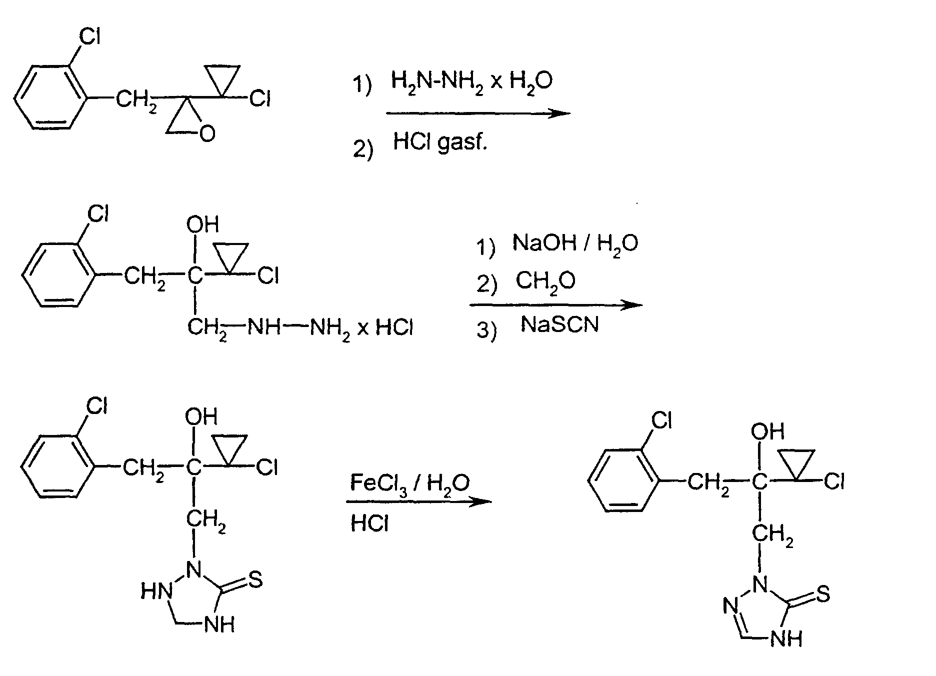

Das bei der Durchführung des erfindungsgemäßen Verfahrens als Ausgangsprodukt benötigte 2-(1-Chlor-cycloprop-1-yl)-2-(2'-chlor-benzyl)-oxiran der Formel (II) ist bekannt (vgl. EP-A 0 297 345). Es lässt sich herstellen, indem man das Chlorhydrin-Derivat der Formel 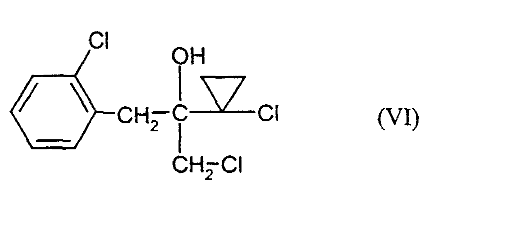 in Gegenwart eines Säurebindemittels, wie Kalium-tert.-butylat, Natriummethylat oder Kaliumcarbonat, und in Gegenwart eines Verdünnungsmittels, wie Dimethylformamid, Methanol, n-Butanol, Tetrahydrofuran, Methyl-tert.-butyl-ether oder Toluol, bei Temperaturen zwischen 20°C und 60°C umsetzt.

Das Oxiran der Formel (II) kann bei der Durchführung der ersten Stufe des erfindungsgemäßen Verfahrens sowohl in reinem Zustand als auch im Gemisch mit dem Chlorhydrin-Derivat der Formel (VI) eingesetzt werden.

Als aromatische Kohlenwasserstoffe kommen bei der Durchführung der ersten Stufe des erfindungsgemäßen Verfahrens vorzugsweise Benzol, Toluol oder Xylol in Frage. Besonders bevorzugt ist der Einsatz von Toluol im Gemisch mit Acetonitril. Dabei ist es ganz besonders vorteilhaft, Acetonitril in äquimolarer Menge zum Oxiran der Formel (II) zu verwenden.

Sowohl bei der Durchführung der ersten Stufe als auch bei der Durchführung der zweiten und dritten Stufe des erfindungsgemäßen Verfahrens arbeitet man im allgemeinen unter Atmosphärendruck. Es ist aber auch möglich, unter erhöhtem Druck oder, sofern keine gasförmigen Komponenten an der Reaktion beteiligt sind, auch unter vermindertem Druck zu arbeiten.

Bei der Durchführung der ersten Stufe des erfindungsgemäßen Verfahrens können die Reaktionstemperaturen innerhalb eines bestimmten Bereiches variiert werden. Im allgemeinen arbeitet man bei Temperaturen zwischen 20°C und 150°C, vorzugsweise zwischen 60°C und 100°C.

Bei der Durchführung der ersten Stufe des erfindungsgemäßen Verfahrens setzt man auf 1 mol an Oxiran der Formel (II) im allgemeinen 3 bis 6 mol an Hydrazinhydrat ein. Im einzelnen verfährt man in der Weise, dass man Oxiran der Formel (II), gegebenenfalls im Gemisch mit Chlorhydrin der Formel (VI) mit Hydrazinhydrat in Gegenwart von Toluol sowie gegebenenfalls in Gegenwart von einer in Bezug auf das Oxiran der Formel (II) äquivalenten Menge an Acetonitril umsetzt. Die Aufarbeitung erfolgt dann nach üblichen Methoden. Im allgemeinen geht man so vor, dass man das Reaktionsgemisch auf Raumtemperatur abkühlt, mit Wasser versetzt, die organische Phase abtrennt, mit Wasser wäscht und dann unter Kühlung eine äquivalente Menge oder auch einen Überschuss an trockenem Chlorwasserstoffgas einleitet. Der anfallende Feststoff wird abgetrennt, mit Toluol, gegebenenfalls im Gemisch mit einem weiteren Kohlenwasserstoff, gewaschen und getrocknet. Es ist jedoch auch möglich, mit wässriger Salzsäure zu extrahieren. Der anfallende Feststoff wird abgetrennt, mit Toluol, gegebenenfalls im Gemisch mit einem weiteren Kohlenwasserstoff, gewaschen und getrocknet.

In einer bevorzugten Variante arbeitet man sowohl bei der Durchführung der ersten als auch der zweiten Stufe des erfindungsgemäßen Verfahrens unter Schutzgasatmosphäre. Als Schutzgas kommt dabei vorzugsweise Argon oder Stickstoff in Betracht.

Als Alkalimetall-hydroxid kommt bei der Durchführung der zweiten Stufe des erfindungsgemäßen Verfahrens vorzugsweise Lithiumhydroxid, Natriumhydroxid oder Kaliumhydroxid in Betracht. Besonders bevorzugt ist der Einsatz von Natriumhydroxid.

Als Verdünnungsmittel kommen bei der Durchführung der zweiten Stufe des erfindungsgemäßen Verfahrens sowohl bei der Behandlung von Hydrazin-hydrochlorid-Derivat der Formel (III) als auch bei der anschließenden Umsetzung mit Formaldehyd und Thiocyanat der Formel (IV) als aromatische Kohlenwasserstoffe vorzugsweise Benzol, Toluol oder Xylol in Frage. Als niedere Alkohole sind Methanol, Ethanol oder Propanol bevorzugt. Als Alkylcarbonsäurealkylester kommt vorzugsweise Essigsäureethylester in Betracht. Besonders bevorzugt führt man sowohl die Neutralisation als auch die weitere Umsetzung in der zweiten Stufe des erfindungsgemäßen Verfahrens entweder in Gegenwart von Toluol im Gemisch mit Ethanol oder in Gegenwart von Essigsäureethylester durch.

Der bei der Durchführung der zweiten Stufe des erfindungsgemäßen Verfahrens als Reaktionskomponente benötigte Formaldehyd kann als Paraformaldehyd, als gasförmiger Formaldehyd oder auch als Formalin-Lösung (= wässrige Formaldehyd-Lösung) eingesetzt werden. Bevorzugt ist die Verwendung von Formalin-Lösung.

Als Thiocyanat kommt bei der Durchführung der zweiten Stufe des erfindungsgemäßen Verfahrens bevorzugt Natriumthiocyanat in Betracht.

Als Katalysatoren kommen bei der Durchführung der zweiten Stufe des erfindungsgemäßen Verfahrens alle für derartige Umsetzungen üblichen Reaktionsbeschleuniger in Frage. Bevorzugt ist der Einsatz von Natriumhydrogensulfat.

Die Reaktionstemperaturen können auch bei der Durchführung der zweiten Stufe des erfindungsgemäßen Verfahrens innerhalb eines bestimmten Bereiches variiert werden. Im allgemeinen arbeitet man sowohl bei der Neutralisation des Hydrazinhydrochlorid-Derivates der Formel (III) als auch bei der weiteren Umsetzung bei Temperaturen zwischen 0°C und 30°C, vorzugsweise zwischen 10°C und 25°C.

Bei der Durchführung der zweiten Stufe des erfindungsgemäßen Verfahrens setzt man auf 1 mol an Hydrazin-hydrochlorid-Derivat der Formel (III) im allgemeinen eine äquivalente Menge oder auch einen Überschuss an Alkalimetallhydroxid, 1 bis 2 mol an Formaldehyd sowie 1 bis 2 mol an Thiocyanat der Formel (IV) und gegebenenfalls 1 bis 2 mol an Natriumhydrogensulfat und Wasser ein, wobei Wasser auch im Überschuss vorhanden sein kann. Die Aufarbeitung erfolgt nach üblichen Methoden. Im allgemeinen geht man so vor, dass man das Reaktionsgemisch mit Wasser versetzt, die organische Phase abtrennt, mit gesättigter, wässriger Kochsalz-Lösung und mit Wasser wäscht, trocknet und einengt. Gegebenenfalls noch vorhandene Verunreinigungen lassen sich nach üblichen Methoden, wie Umkristallisation, entfernen.

Als inerte organische Verdünnungsmittel kommen bei der Durchführung der dritten Stufe des erfindungsgemäßen Verfahrens vorzugsweise Ethanol, Essigsäureethylester oder Gemische aus Ethanol und Toluol in Frage.

Die Reaktionstemperaturen können auch bei der Durchführung der dritten Stufe des erfindungsgemäßen Verfahrens in einem bestimmten Bereich variiert werden. Im allgemeinen arbeitet man bei Temperaturen zwischen 0°C und 100°C, vorzugsweise zwischen 10°C und 65°C.

Bei der Durchführung der dritten Stufe des erfindungsgemäßen Verfahrens setzt man auf 1 mol an Triazolidin-Verbindung der Formel (V) im allgemeinen eine äquivalente Menge oder auch einen Überschuss an Eisen (III)-chlorid ein. Die Aufarbeitung erfolgt nach üblichen Methoden. Im allgemeinen verfährt man in der Weise, dass man gegebenenfalls nach vorherigem Versetzen des Reaktionsgemisches mit Wasser die Phasen trennt, die organische Phase wäscht, trocknet und einengt. Gegebenenfalls noch vorhandene Verunreinigungen lassen sich nach üblichen Methoden, zum Beispiel durch Umkristallisation, entfernen.

In einer besonderen Variante kann das erfindungsgemäße Verfahren in der Weise durchgeführt werden, dass die Stufen zwei und drei als Eintopfreaktion vorgenommen werden. Dabei wird das in der zweiten Stufe anfallende Gemisch ohne vorherige Isolierung des Triazolidins der Formel (V) direkt der Oxidation in der dritten Stufe unterworfen. Vor Zugabe der Eisen (III)-chlorid-Lösung muss das in der zweiten Stufe anfallende Reaktionsgemisch allerdings gründlich mit Wasser gewaschen werden, um eventuell vorhandenes, überschüssiges Thiocyanat zu entfernen.

Das erfindungsgemäß herstellbare Triazolinthion-Derivat kann in der "Thiono"-Form der Formel 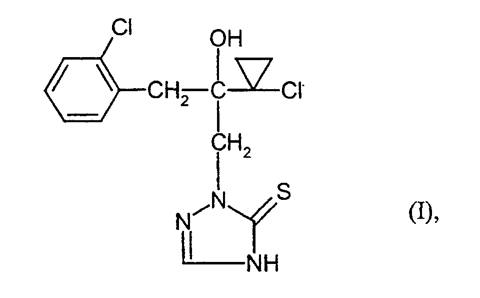 oder in der tautomeren "Mercapto"-Form der Formel 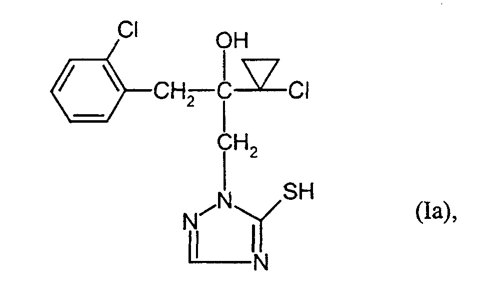 vorliegen. Der Einfachheit halber wird jeweils nur die "Thiono"-Form aufgeführt.

Das erfindungsgemäß herstellbare Triazolinthion-Derivat ist als Wirkstoff mit mikrobiziden, insbesondere fungiziden Eigenschaften bekannt (vgl. WO 96-16 048).

Die Durchführung des erfindungsgemäßen Verfahrens wird durch die folgenden Beispiele veranschaulicht.

### Herstellungsbeispiele

### Beispiel 1

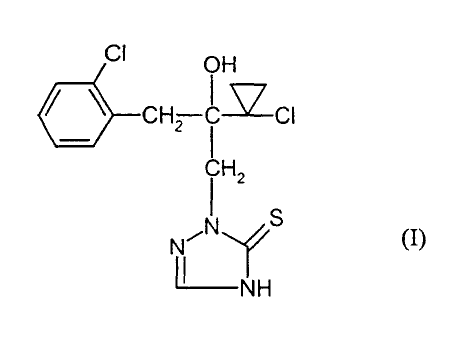

a) Herstellung der Verbindung der Formel 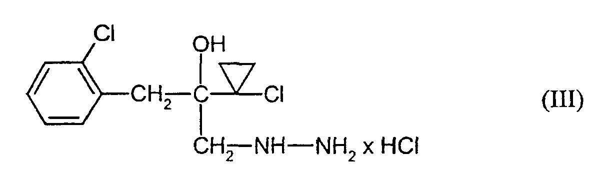
   In ein Gemisch aus 25 ml (0,5 mol) Hydrazinhydrat und 5,2 ml (0,1 mol) Acetonitril werden unter Rühren bei Raumtemperatur und unter Argonatmosphäre 174 g einer Lösung von 0,1 mol 2-(1-Chlor-cycloprop-1-yl)-2-(2'chlor-benzyl)-oxiran in Toluol gegeben. Das Reaktionsgemisch wird unter intensivem Rühren auf 85°C erhitzt und 4 Stunden bei dieser Temperatur belassen. Danach lässt man auf Raumtemperatur abkühlen, gibt 40 ml Wasser hinzu und trennt die Phasen.
   Die organische Phase wird zweimal mit je 20 ml Wasser gewaschen. Anschließend werden unter Kühlung mit Trockeneis 1,5 Äquivalente trockenes Chlorwasserstoffgas eingeleitet. Nach beendeter Zugabe wird 16 Stunden bei Raumtemperatur gerührt. Der anfallende kristalline Feststoff wird abgetrennt, mit wenig Toluol und Petrolether gewaschen und getrocknet. Man erhält auf diese Weise 30,5 g eines Produktes, das gemäß HPLC zu 97,9 % aus 2-(1-Chlor-cycloprop-1-yl)-3-(2-chlor-phenyl)-2-hydroxy-propyl-1-hydrazin-hydrochlorid besteht. Die Ausbeute errechnet sich danach zu 95,9 % der Theorie.
b) Herstellung der Verbindung der Formel 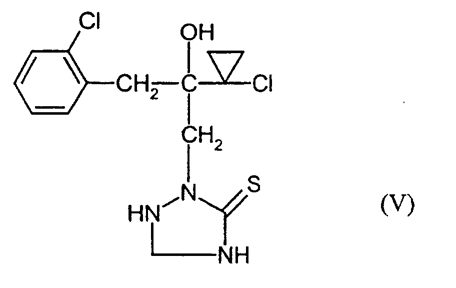
   Ein Gemisch aus 93,4 g (0,3 mol) 2-(1-Chlor-cycloprop-1-yl)-3-(2-chlorphenyl)-2-hydroxy-propyl-1-hydrazin-hydrochlorid und 1000 ml Essigsäureethylester wird unter Rühren bei Raumtemperatur und unter Argonatmosphäre mit 12,0 g (0,3 mol) Natriumhydroxid-Micropills und 20 ml Wasser versetzt. Das Reaktionsgemisch wird 1 Stunde bei Raumtemperatur gerührt und dann mit 20,8 ml (0,276 mol Formaldehyd) Formalin-Lösung (36,5 %ig in Wasser) versetzt. Nach 30-minütigem Rühren bei Raumtemperatur werden 23,8 g (0,294 mol) Natriumthiocyanat und 63,0 g (0,524 mol) Natriumhydrogensulfat hinzugegeben, und es wird weitere 2 Stunden bei Raumtemperatur gerührt. Anschließend fügt man 300 ml Wasser hinzu und trennt die Phasen. Die organische Phase wird zweimal mit gesättigter, wässriger Kochsalz-Lösung und mit Wasser gewaschen, dann über Natriumsulfat getrocknet und unter vermindertem Druck eingeengt. Man erhält auf diese Weise 119,4 g eines Feststoffes, der gemäß HPLC zu 80,35 % aus 2-(1-Chlor-cycloprop-1-yl)-1-(2-chlor-phenyl)-2-hydroxy-3-(1,2,4-triazolidin-5-thiono-1-yl)-propan besteht. Die Ausbeute errechnet sich danach zu 94,8 % der Theorie.
c) Herstellung der Verbindung der Formel 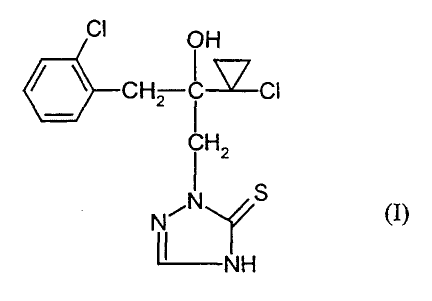
   Ein Gemisch aus 1,8 g (0,005 mol) 2-(1-Chlor-cycloprop-1-yl)-1-(2-chlorphenyl)-2-hydroxy-3-(1,2,4-triazolidin-5-thiono-1-yl)-propan, 40 ml Toluol und 10 ml Ethanol wird bei Raumtemperatur und unter Rühren mit 20 ml (0,01 mol) 0,5-molarer wässriger, leicht salzsaurer Eisen (III)-chlorid-Lösung versetzt. Man rührt das Reaktionsgemisch 6 Stunden bei Raumtemperatur und trennt dann die Phasen. Die organische Phase wird zweimal mit Wasser und gesättigter, wässriger Kochsalz-Lösung gewaschen, über Natriumsulfat getrocknet und unter vermindertem Druck eingeengt. Man erhält auf diese Weise 1,8 g Feststoff, der gemäß HPLC zu 94,8 % aus 2-(1-Chlor-cycloprop-1-yl)-1-(2-chlor-phenyl)-3-(4,5-dihydro-1,2,4-triazol-5-thiono-1-yl)-propan-2-ol besteht. Die Ausbeute errechnet sich danach zu 99,2 % der Theorie.

### Beispiel 2

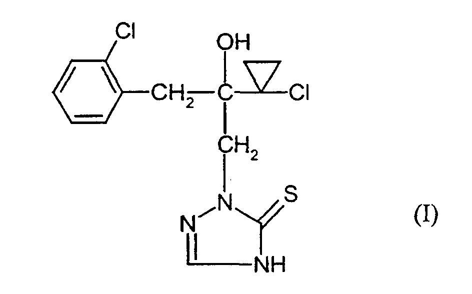

### 3. Stufe

Ein Gemisch aus 1,8 g (0,005 mol) 2-(1-Chlor-cycloprop-1-yl)-1-(2-chlor-phenyl)-2-hydroxy-3-(1,2,4-triazolidin-5-thiono-1-yl)-propan und 50 ml Ethanol wird bei Raumtemperatur unter Rühren und unter Kühlung mit 20 ml (0,01 mol) 0,5-molarer wässriger, leicht salzsaurer Eisen (III)-chlorid-Lösung versetzt. Man rührt weitere 2 Stunden bei Raumtemperatur, gibt das Reaktionsgemisch dann auf Eiswasser und extrahiert mit Essigsäureethylester. Die organische Phase wird zweimal mit Wasser und gesättigter, wässriger Kochsalz-Lösung gewaschen, über Natriumsulfat getrocknet und unter vermindertem Druck eingeengt. Man erhält auf diese Weise 1,74 g Feststoff, der gemäß HPLC zu 97,1 % aus 2-(1-Chlor-cycloprop-1-yl)-1-(2-chlorphenyl)-3-(4,5-dihydro-1,2,4-triazol-5-thiono-1-yl)-propan-2-ol besteht. Die Ausbeute errechnet sich danach zu 98,2 % der Theorie.

### Vergleichsbeispiel A

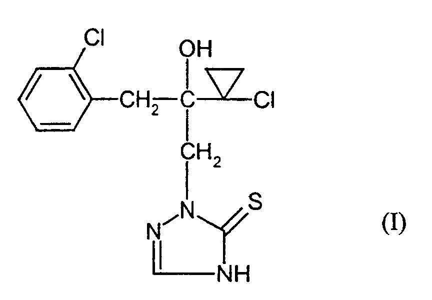

a) Herstellung der Verbindung der Formel 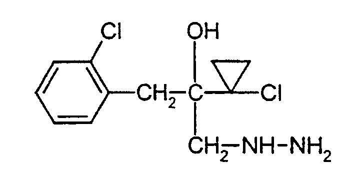
   Ein Gemisch aus 27,8 g (0,1 mol) 3-Chlor-2-(1-chlor-cycloprop-1-yl)-1-(2-chlor-phenyl)-propan-2-ol und 48,5 ml (1 mol) Hydrazinhydrat wird unter Stickstoffatmosphäre und unter Rühren 5 Stunden auf 100°C erhitzt. Nach dem Abkühlen des Zweiphasensystems auf Raumtemperatur wird die Hydrazin-Phase abdekantiert und der Rückstand einmal mit 20 ml Wasser gewaschen. Dabei verbleiben 25,9 g eines Produktes, das gemäß gaschromatographischer Analyse zu 86,8 % aus 2-(1-Chlor-cycloprop-1-yl)-3-(2-chlorphenyl)-2-hydroxy-propyl-1-hydrazin besteht. Die Ausbeute errechnet sich danach zu 94,5 % der Theorie. Nach Umkristallisation des Rohproduktes aus Acetonitril erhält man [2-(1-Chlor-cycloprop-1-yl)-3-(2-chlor-phenyl)-2-hydroxy]-propyl-1-hydrazin in Form einer Festsubstanz vom Schmelzpunkt 86°C bis 88°C.
b) Herstellung der Verbindung der Formel 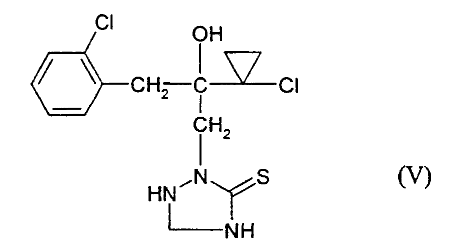
   Ein Gemisch aus 5,48 g (20 mmol) [2-(1-Chlor-cycloprop-1-yl)-3-(2-chlorphenyl)-2-hydroxy]-propyl-1-hydrazin, 40 ml Methyl-tert.-butylether, 0,9 g (30 mmol) Paraformaldehyd und 1,84 g (24 mmol) Ammoniumthiocyanat wird 3 Stunden unter Rühren auf 60°C erhitzt. Nach dem Abkühlen auf Raumtemperatur wird das Reaktionsgemisch mit Methyl-tert.-butylether verdünnt und mit gesättigter, wässriger Natriumcarbonat-Lösung gewaschen. Die organische Phase wird über Natriumsulfat getrocknet, filtriert und unter vermindertem Druck eingeengt. Man erhält auf diese Weise 6,1 g eines Produktes, das gemäß HPLC-Analyse zu 86,9 % aus 2-(1-Chlor-cycloprop-1-yl)-1-(2-chlorphenyl)-2-hydroxy-3-(1,2,4-triazolidin-5-thiono-1-yl)-propan besteht. Nach Zugabe von wenig Dichlormethan fällt 2-(1-Chlor-cycloprop-1-yl)-1-(2-chlor-phenyl)-2-hydroxy-3-(1,2,4-triazolidin-5-thiono-1-yl)-propan in Form einer kristallinen Festsubstanz aus.
c) Herstellung der Verbindung der Formel 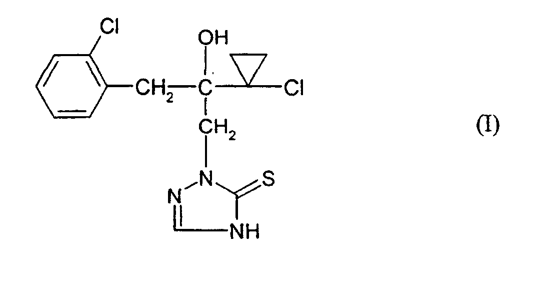
   Über ein auf 70°C erwärmtes Gemisch aus 1,72 g (5 mmol) 2-(1-Chlorcycloprop-1-yl)-1-(2-chlor-phenyl)-2-hydroxy-3-(1,2,4-triazolidin-5-thiono-1-yl)-propan, 10 ml absolutem Toluol, 0,34 g (6 mmol) Kaliumhydroxid-Pulver und 10 mg Schwefel-Pulver wird unter Rühren 3,5 Stunden lang ein Luftstrom geleitet. Dabei wird der Fortgang der Umsetzung durch HPLC-Analyse kontrolliert. Nach dem Abkühlen auf Raumtemperatur wird das Reaktionsgemisch mit Methyl-tert.-butylether verdünnt und mehrfach mit gesättigter, wässriger Ammoniumchlorid-Lösung gewaschen. Die organische Phase wird über Natriumsulfat getrocknet, filtriert und unter vermindertem Druck eingeengt. Man erhält auf diese Weise 2,2 g eines Produktes, das gemäß HPLC-Analyse zu 71 % aus 2-(1-Chlor-cycloprop-1-yl)-1-(2-chlor-phenyl)-3-(4,5-dihydro-1,2,4-triazol-5-thiono-1-yl)-propan-2-ol besteht.

## Patentansprüche

1. Verfahren zur Herstellung von 2-(1-Chlor-cycloprop-1-yl)-1-(2-chlor-phenyl)-3-(4,5-dihydro-1,2,4-triazol-5-thiono-1-yl)-propan-2-ol der Formel 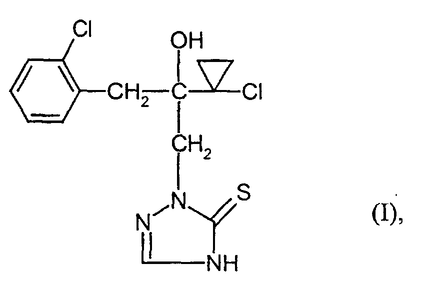 **dadurch gekennzeichnet, dass** man
a) in einer ersten Stufe 2-(1-Chlor-cycloprop-1-yl)-2-(2'-chlor-benzyl)-oxiran der Formel 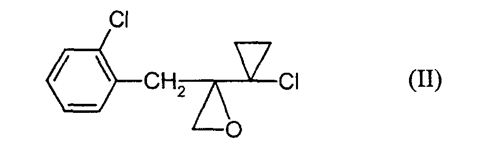 zunächst mit Hydrazinhydrat in Gegenwart von aromatischem Kohlenwasserstoff, gegebenenfalls im Gemisch mit Acetonitril, umsetzt und dann Chlorwasserstoff einleitet, oder mit wässriger Salzsäure extrahiert,
b) danach in einer zweiten Stufe das entstandene 2-(1-Chlor-cycloprop-1-yl)-3-(2-chlor-phenyl)-2-hydroxy-propyl-1-hydrazin-hydrochlorid der Formel 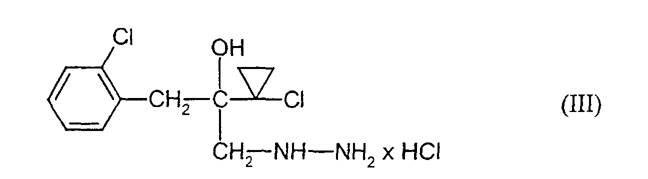 mit Alkalimetall-hydroxid in Gegenwart von Wasser sowie in Gegenwart von aromatischem Kohlenwasserstoff, gegebenenfalls im Gemisch mit einem niederen Alkohol, oder in Gegenwart von Alkylcarbonsäurealkylester behandelt und dann nacheinander mit Formaldehyd und Thiocyanat der Formel
X-SCN (IV),
in welcher
X für Natrium, Kalium oder Ammonium steht,
in Gegenwart von Wasser sowie in Gegenwart von aromatischem Kohlenwasserstoff, gegebenenfalls im Gemisch mit einem niederen Alkohol, oder in Gegenwart von Alkylcarbonsäurealkylester sowie gegebenenfalls in Gegenwart eines Katalysators umsetzt und
c) anschließend in einer dritten Stufe das entstandene 2-(1-Chlorcycloprop-1-yl)-1-(2-chlor-phenyl)-2-hydroxy-3-(1,2,4-triazolidin-5-thiono-1-yl)-propan der Formel 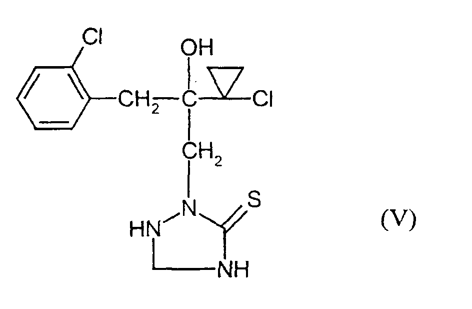 mit Eisen (III)-chlorid in Gegenwart von wässriger Salzsäure sowie in Gegenwart eines inerten organischen Verdünnungsmittels umsetzt.

2. Verfahren gemäß Anspruch 1, **dadurch gekennzeichnet, dass** man bei der Durchführung der ersten Stufe Toluol im Gemisch mit Acetonitril als Verdünnungsmittel einsetzt.

3. Verfahren gemäß Anspruch 1, **dadurch gekennzeichnet, dass** man bei der Durchführung der zweiten Stufe Natriumhydroxid als Neutralisationsmittel einsetzt.

4. Verfahren gemäß Anspruch 1, **dadurch gekennzeichnet, dass** man bei der Durchführung der zweiten Stufe Essigsäureethylester oder Toluol im Gemisch mit Ethanol als Verdünnungsmittel einsetzt.

5. Verfahren gemäß Anspruch 1, **dadurch gekennzeichnet, dass** man bei der Durchführung der zweiten Stufe den Formaldehyd in Form von Formalin-Lösung einsetzt.

6. Verfahren gemäß Anspruch 1, **dadurch gekennzeichnet, dass** man bei der Durchführung der zweiten Stufe Natriumthiocyanat als Reaktionskomponente und Natriumhydrogensulfat als Katalysator einsetzt.

7. Verfahren gemäß Anspruch 1, **dadurch gekennzeichnet, dass** man bei der Durchführung der dritten Stufe Essigsäureethylester, Ethanol oder ein Gemisch aus Toluol und Ethanol als Verdünnungsmittel einsetzt.

8. Verfahren gemäß Anspruch 1, **dadurch gekennzeichnet, dass** man bei der Durchführung der ersten Stufe bei Temperaturen zwischen 20°C und 150°C arbeitet.

9. Verfahren gemäß Anspruch 1, **dadurch gekennzeichnet, dass** man bei der Durchführung der zweiten Stufe bei Temperaturen zwischen 0°C und 30°C arbeitet.

10. Verfahren gemäß Anspruch 1, **dadurch gekennzeichnet, dass** man bei der Durchführung der dritten Stufe bei Temperaturen zwischen 0°C und 100°C arbeitet.

## Claims

1. Process for preparing 2-(1-chloro-cycloprop-1-yl)-1-(2-chloro-phenyl)-3-(4,5-dihydro-1,2,4-triazole-5-thiono-1-yl)-propan-2-ol of the formula 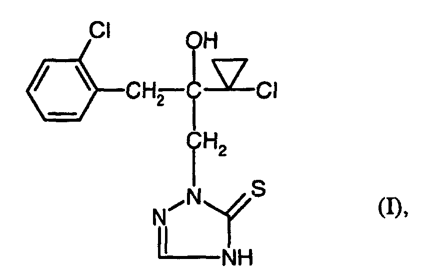 **characterized in that**
a) in a first step, 2-(1-chloro-cycloprop-1-yl)-2-(2'-chloro-benzyl)-oxirane of the formula 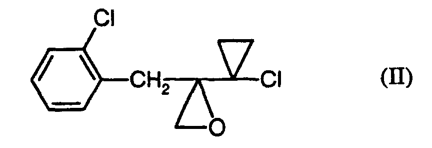 is initially reacted with hydrazine hydrate in the presence of aromatic hydrocarbon, if appropriate in a mixture with acetonitrile, and hydrogen chloride is then introduced, or the mixture is extracted with aqueous hydrochloric acid
b) in a second step, the resulting 2-(1-chloro-cycloprop-1-yl)-3-(2-chloro-phenyl)-2-hydroxy-propyl-1-hydrazine hydrochloride of the formula 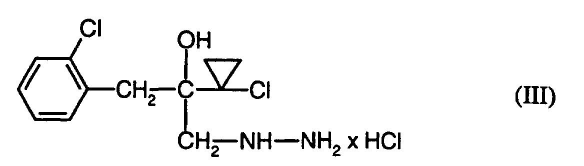 is then treated with alkali metal hydroxide in the presence of water and in the presence of aromatic hydrocarbon, if appropriate in a mixture with a lower alcohol, or in the presence of alkyl alkylcarboxylate, and then reacted successively with formaldehyde and thiocyanate of the formula
X-SCN (IV),
in which
X represents sodium, potassium or ammonium,
in the presence of water and in the presence of aromatic hydrocarbon, if appropriate in a mixture with a lower alcohol, or in the presence of alkyl alkylcarboxylate and, if appropriate, in the presence of a catalyst, and
c) in a third step, the resulting 2-(1-chloro-cycloprop-1-yl)-1-(2-chloro-phenyl)-2-hydroxy-3-(1,2,4-triazolidine-5-thiono-1-yl)-propane of the formula 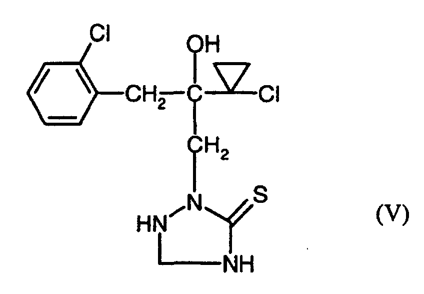 is then reacted with iron(III) chloride in the presence of aqueous hydrochloric acid and in the presence of an inert organic diluent.

2. Process according to claim 1, **characterized in that** the diluent used for carrying out the first step is toluene in a mixture with acetonitrile.

3. Process according to Claim 1, **characterized in that** the neutralizing agent used for carrying out the second step is sodium hydroxide.

4. Process according to Claim 1, **characterized in that** the diluent used for carrying out the second step is ethyl acetate or toluene in a mixture with ethanol.

5. Process according to Claim 1, **characterized in that** the formaldehyde used for carrying out the second step is employed in the form of Formalin solution.

6. Process according to Claim 1, **characterized in that** the reaction component and the catalyst used for carrying out the second step are sodium thiocyanate and sodium hydrogen sulphate, respectively.

7. Process according to Claim 1, **characterized in that** the diluent used for carrying out the third step is ethyl acetate, ethanol or a mixture of toluene and ethanol.

8. Process according to Claim 1, **characterized in that** the first step is carried out at temperatures between 20°C and 150°C.

9. Process according to Claim 1, **characterized in that** the second step is carried out at temperatures between 0°C and 30°C.

10. Process according to Claim 1, **characterized in that** the third step is carried out at temperatures between 0°C and 100°C.

## Revendications

1. Procédé de préparation du 2-(1-chloro-cycloprop-1-yl)-1-(2-chlorophényl)-3-(4,5-dihydro-1,2,4-triazole-5-thiono-1-yl)propane-2-ol de la formule : 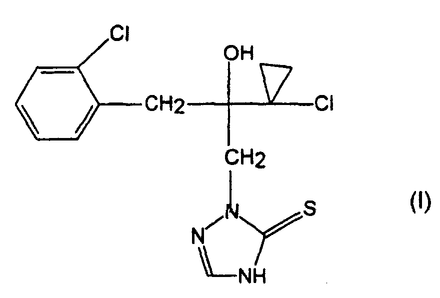 **caractérisé en ce que** :
a) dans une première étape, on fait réagir le 2-(1-chlorocycloprop-1-yl)-2-(2'-chlorobenzyl)oxiranne de la formule : 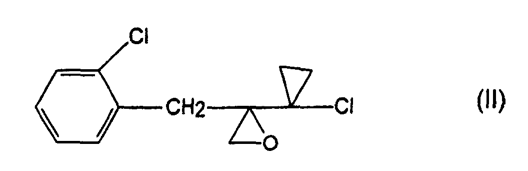 d'abord avec l'hydrate d'hydrazine en présence d'un hydrocarbure aromatique, le cas échéant en mélange avec l'acétonitrile, puis on fait passer du chlorure d'hydrogène, ou on extrait avec de l'acide chlorhydrique aqueux,
b) puis, dans une deuxième étape, on traite le chlorhydrate du 2-(1-chlorocycloprop-1-yl)-3-(2-chlorophényl)-2-hydroxypropyl-1-hydrazine de la formule : 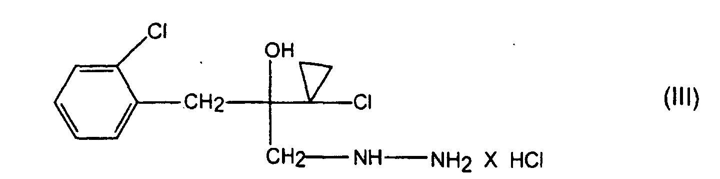 avec un hydroxyde de métal alcalin en présence d'eau ainsi qu'en présence d'un hydrocarbure aromatique, le cas échéant en présence d'un alcool inférieur, ou en présence d'un ester alcoylique d'acide alcoylcarboxylique, ensuite on fait réagir successivement avec le formaldéhyde et le thiocyanate de la formule :
X-SCN (IV)
dans laquelle:
X représente l'atome de sodium, de potassium ou le radical ammonium,
en présence d'eau ainsi qu'en présence d'un hydrocarbure aromatique, le cas échéant en mélange avec un alcool inférieur, ou en présence d'un ester alcoylique d'acide alcoylcarboxylique, ainsi que le cas échéant, en présence d'un catalyseur, et
c) ensuite, dans une troisième étape, le 2-(1-chlorocycloprop-l-yl)-1-(2-chlorophényl)-2-hydroxy-3-(1,2,4-triazolidine-5-thiono-1-yl)propane de la formule : 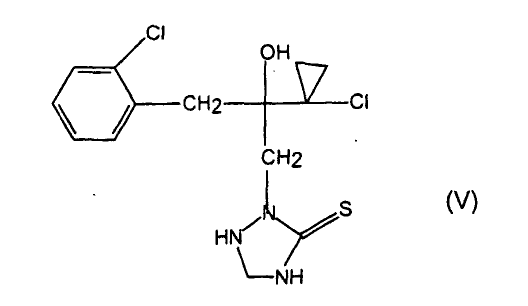 avec du chlorure de fer (III) en présence d'acide chlorhydrique aqueux, ainsi qu'en présence d'un agent de dilution organique inerte.

2. Procédé suivant la revendication 1, **caractérisé en ce que** l'on met en oeuvre lors de la réalisation de la première étape, du toluène en mélange avec de l'acétonitrile comme agent de dilution.

3. Procédé suivant la revendication 1, **caractérisé en ce que** l'on met en oeuvre lors de la réalisation de la deuxième étape, de l'hydroxyde de sodium comme agent de neutralisation.

4. Procédé suivant la revendication 1, **caractérisé en ce que** l'on met en oeuvre lors de la réalisation de la deuxième étape, de l'acétate d'éthyle ou du toluène en mélange avec de l'éthanol comme agent de dilution.

5. Procédé suivant la revendication 1, **caractérisé en ce que** l'on met en oeuvre lors de la réalisation de la deuxième étape, du formaldéhyde sous la forme d'une solution de formaline.

6. Procédé suivant la revendication 1, **caractérisé en ce que** l'on met en oeuvre lors de la réalisation de la deuxième étape, du thiocyanate de sodium comme composant de réaction et de l'hydrogénosulfate de sodium comme catalyseur.

7. Procédé suivant la revendication 1, **caractérisé en ce que** l'on met en oeuvre lors de la réalisation de la troisième étape, de l'acétate d'éthyle, de l'éthanol ou un mélange de toluène et d'éthanol comme agent de dilution.

8. Procédé suivant la revendication 1, **caractérisé en ce que** l'on travaille lors de la réalisation de la première étape, à des températures allant de 20°C à 150°C.

9. Procédé suivant la revendication 1, **caractérisé en ce que** l'on travaille lors de la réalisation de la deuxième étape, à des températures allant de 0°C à 30°C.

10. Procédé suivant la revendication 1, **caractérisé en ce que** l'on travaille lors de la réalisation de la troisième étape, à des températures allant de 0°C à 100°C.
